## Europäisches Patentamt

## European Patent Office

## Office européen des brevets

(19)

(11) Veröffentlichungsnummer: **0 064 970**
**B1**

(12) # EUROPÄISCHE PATENTSCHRIFT

(45) Veröffentlichungstag der Patentschrift:
**23.07.86**

(51) Int. Cl.⁴: **A 61 B 5/00,** G 01 N 27/26,
G 01 N 27/02

(21) Anmeldenummer: **82890066.2**

(22) Anmeldetag: **05.05.82**

(54) **Medizinische Sonde.**

(30) Priorität: **07.05.81 AT 2029/81**

(43) Veröffentlichungstag der Anmeldung:
**17.11.82 Patentblatt 82/46**

(45) Bekanntmachung des Hinweises auf die Patenterteilung:
**23.07.86 Patentblatt 86/30**

(84) Benannte Vertragsstaaten:
**BE CH DE FR GB IT LI LU NL SE**

(56) Entgegenhaltungen:
**EP-A-0 023 157**
**AT-B-351 848**
**DE-A-2 943 958**
**FR-A-2 327 539**
**FR-A-2 360 074**
**FR-A-2 425 641**

(73) Patentinhaber: **OTTOSENSORS CORPORATION,**
**3440 Wilshire Boulevard - Suite 1010, Los**
**Angeles, CA 90010 (US)**

(72) Erfinder: **Prohaska, Otto, Dipl.- Ing. Dr., Prinz-**
**Eugenstrasse 76, A-1040 Wien (AT)**

(74) Vertreter: **Wildhack, Helmut, Dipl.- Ing. Dr.,**
**Patentanwälte Dipl.- Ing. Dr. H. Collin, Dipl.-**
**Ing. E. Buresch, Dipl.- Ing. Dr. H. Wildhack,**
**Dipl.- Ing. A. Häupl Mariahilferstrasse 50, A-1070**
**Wien (AT)**

LIBER, STOCKHOLM 1986

## Beschreibung

Die Erfindung bezieht sich auf eine medizinische Sonde in Dünnschichtausführung, insbesondere zur Registrierung bzw. Beeinflussung von Gewebeaktivitäten, mit einem isolierenden Substrat, auf welchem Leiterbahnen angeordnet sind, welche gegenüber dem Meßgut mittels einer ersten Isolierschichtabdeckung z.B. aus $SiO_2$ isoliert sind und zu auf dem isolierenden Substrat aufliegenden Elektroden- bzw. Geberflächen führen.

Aus der AT-PS 351 848 ist eine Sonde der obigen Art bekanntgeworden, die insbesondere zur Registrierung bzw. Beeinflussung von Zellaktivitäten dient und ein Substrat, auf diesem angeordnete Leiterbahnen, welche zu Sonden- bzw. Geberflächen führen, und eine darüber angeordnete Isolierschicht aufweist. Dabei ist die Isolierschicht auf das Substrat mit den Leiterbahnen aufgedampft und besitzt vorzugsweise eine Dicke von 0,1 bis 5 μm. Auf der Isolierschicht ist eine Schicht eines elektrolytisch oder thermisch oxydierbaren Metalls angeordnet. Die Herstellung und der Aufbau dieser Sonde sind aufwendig.

Aus der AT-PS 342 189 ist eine medizinische Elektrode bekannt geworden, die aus einem Körper aus nicht leitendem Material besteht, der an einem Ende die Kontaktelektrode trägt, welche mit einer den Körper durchsetzenden Zuleitung verbunden ist, wobei weiters über die wiederholt verwendbare Elektrode eine wegwerfbare Einmalhülle aus nicht leitendem Material angebracht ist, die in ihrem Inneren jedoch einen Hohlraum freiläßt, welcher an die Kontaktelektrode grenzt und über zumindest eine Öffnung in der Einmalhülle mit der Außenfläche in Verbindung steht und mit einem zähflüssigen Elektrolyten gefüllt ist.

Besonders bedeutungsvoll sind solche als Dünnschichtsonden bezeichnete Sonden bzw. Geber in der medizinischen Forschung wie auch in der Humanmedizin, um elektrische Gewebeaktivitäten, Stoffwechselabläufe, Durchblutung, Ionen- und Molekülhaushalt od.dgl. zu bestimmen bzw. zu beeinflussen.

Dünnschichtsonden eignen sich besonders für Vielfachmessungen, da das Herstellungsverfahren große Variabilität in Dimensionierung und Gestaltung erlaubt (O. Prohaska, F. Pacha, P. Pfundner, H. Petsche: A 16-fold semimicroelectrode for intracortical recording of field potentials, Electroenceph. Clin. Neurophysiol., 47, 629-631, 1979).

Die Schwierigkeit besteht aber darin, daß der unmittelbare Kontakt zwischen Gewebe und Metall- bzw. Metallverbindungselektroden und/oder -gebern bei den bekannten Sonden einige wesentliche Nachteile bringt:

a) die Elektrodenübergangsimpedanz zwischen Gewebe und Elektrode ist nicht nur frequenzsondern auch flächenabhängig (H.J.Vetter: Elektrochemische Kinetik, Springer Verl. Berlin, 1961) und limitiert daher die technologisch mögliche Elektrodenminiaturisierung,

b) polarographische Messungen (I.M.Kolthoff, J.J.Lingane: Rolarographie, Intersci. Publ. NY., 1952) verursachen einen Stromfluß durch das Gewebe, wobei eine empfindliche Störung, wenn nicht gar Schädigung des Gewebes bewirkt wird,

c) der extrazelluläre Ionen- und Molekülhaushalt kann nur unter bestimmten Einschränkungen oder überhaupt nicht bestimmt oder beeinflußt werden und

d) Ionenkonzentrationsänderungen im Gewebe können Potentialmessungen stark beeinflussen; z.B. bewirkt die C1⁻ Ionenkonzentrationsänderung eine Elektrodenpotentialänderung an der Ag/AgCl Elsktrode.

Bei einer Sonde der eingangs genannten Art werden diese Nachteile gemäß der Erfindung dadurch beseitigt, daß mindestens eine Elektroden- bzw. Geberfläche von einer auf dem isolierenden Substrat aufgebrachten zweiten Isolierschichtabdeckung, die vorzugsweise eine Wandstärks von 0,5 μm bis 10 μm, insbesondere von 3 μm, aufweist, unter Bildung einer, mit einem gasförmigen und/oder flüssigen und/oder festen Medium gefüllten Kammer überdacht ist, die mindestens eine in Bezug auf die Größe der Kammer kleine Öffnung aufweist, wobei als Stoffe für die zweite Isolierschichtabdeckung $SiO_2$, $SiO_x$, $Si_3N_4$, $SiO_xN_y$, $TiO_2$, $Ta_2O_5$ oder mehrere dieser Stoffe eingesetzt sind.

Bevorzugte Ausbildungen der erfindungsgemäßen medizinischen Sonde sind in den abhängigen Ansprüchen angegeben.

Die wesentlichen, neuen Effekte werden dadurch erzielt, daß Kammern gebildet werden, die die Geberflächen überdachen und ein Medium enhalten, das den Kontakt zwischen Elektroden- bzw. Geberflächen und Meßgut (z.B. Gewebe) herstellt, sowie durch die Vielfachausführung der Kammern auf einer Sonde, wobei die Miniaturisierung, bedingt durch die Dünnschichtausführung, von entscheidender Bedeutung ist.

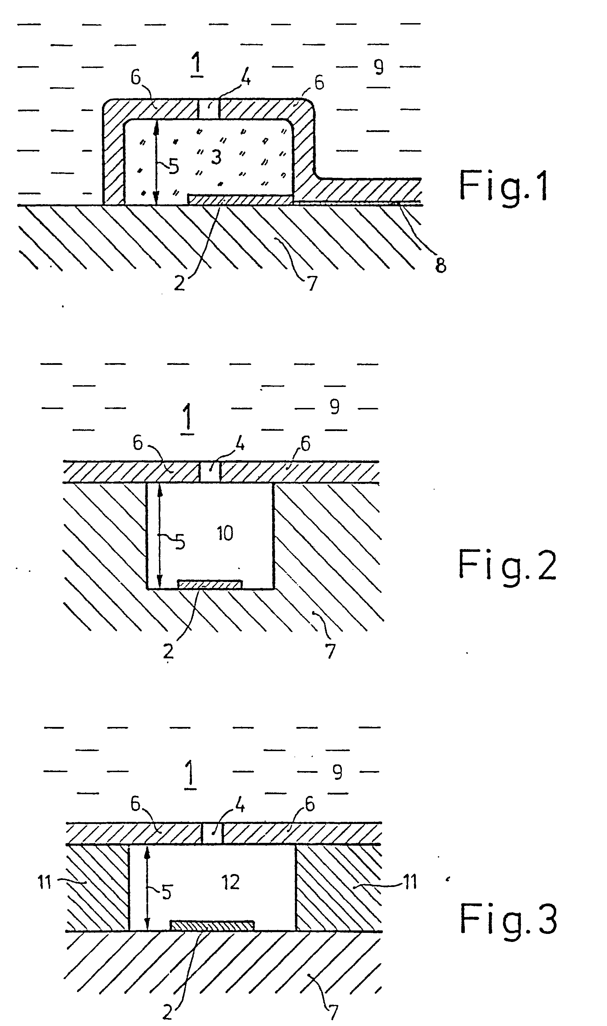

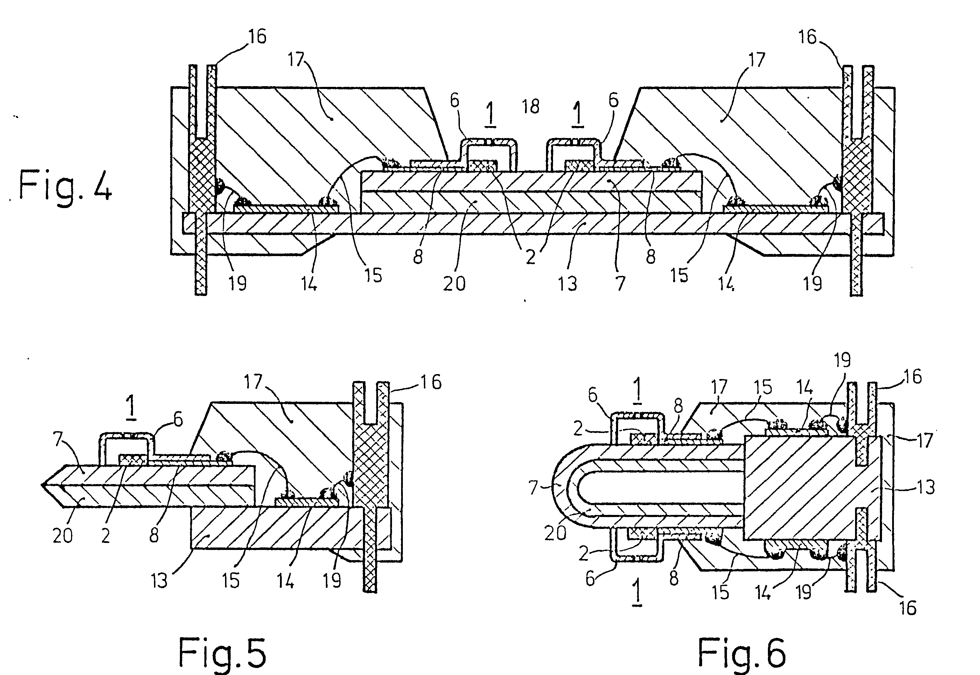

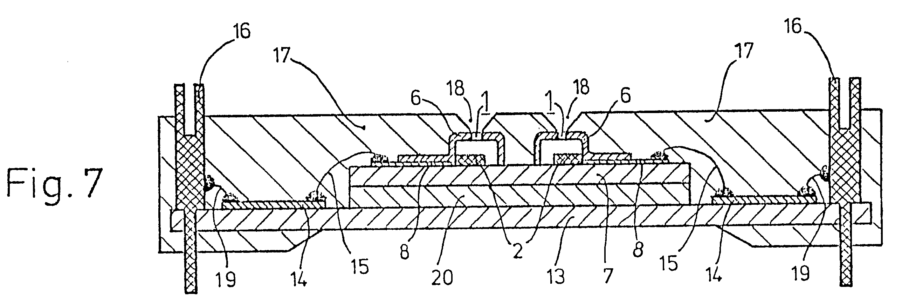

Bezug nehmend auf die Abbildungen wird die Erfindung im folgenden beschrieben: die Fig. 1, 2 und 3 stellen drei mögliche Ausführungsformen der erfindungsgemäßen Kammer 1 dar, die Fig. 4, 5 und 6 zeigen mögliche Sondenausführungen im Schnitt, die Fig. 7 zeigt sine Variante der Fig. 4.

Fig. 1 zeigt eine erhabene, nachfolgend nur mehr als "Dünnschichtpufferbehälter" bezeichnste Kammer 1, gebildet aus einer dünnen Isolierschicht 6, die die äußere Form des Dünnschichtpufferbehälters 1 bestimmt, eine Dicke im Bereich von 0,5 μm bis 10 μm, vorzugsweise 3 μm, hat und wenigstens aus einem der folgenden Stoffe $SiO_2$, $SiO_x$, $Si_3N_4$, $SiO_xN_y$, $TiO_2$, $Ta_2O_5$ besteht und direkt auf dem isolieranden Substrat 7 aufgebracht wird, so daß die dünne Isolierschichtabdeckung 6 mindestens eine Elektroden bzw. Geberfläche 2 überdacht und mindestens eine Öffnung 4 zum Meßgut 9 hin aufweist. Ein neuer Effekt wurde dadurch erzielt, daß die Öffnungen 4 die eigentlichen Meß bzw. Geberflächen der Sonde darstellen, da sie die

-ekte Verbindung zum Meßgut 9 bilden und durch die miniaturisierte Ausführung eine punktförmige, lokale Messung bzw. Beeinflussung ermöglichen. Demgegenüber können die früher die Eigenschaften der Sonde bestimmenden Metall- bzw. Metallverbindungselektroden- bzw. -geberflächen relativ größer gemacht werden, da sie nun nicht mehr unmittelbar mit dem Meßgut 9 sondern erst indirekt über das im Dünnschichtpufferbehälter 1 befindliche Medium 3 mit dem Meßgut 9 in Verbindung stehen. Dadurch kann der elektrodenimpedanzbedingte Meßwertsverzerrungsfaktor beispielsweise bis um den Faktor 1000 gesenkt werden.

Die Art und Form des isolierenden Substrates 7 kann in weitem Rahmen variiert werden: es kann beispielsweise steif oder biegsam, rund, uneben oder eben, flach, nadelförmig oder zylinderförmig, etc. sein.

Fig. 2 zeigt eine andere mögliche Ausführungsform des Dünnschichtpufferbehälters 1, wobei die Behälterform durch Einätzen einer Vertiefung 10 in das isolierende Substrat 7 erreicht wird. In Fig. 3 wird die Behälterform in Schichtbauweise aufgebaut und zwar durch Einätzen einer Vertiefung 12 in einer auf das isolierende Substrat 7 aufgebrachte Isolierschicht 11. In beiden Ausführungsformen von Fig. 2 und 3 werden die Vertiefungen 10 bzw. 12 durch eine dünne Isolierschichtabdeckung 6, in die günstigerweise die Öffnung 4 eingeätzt wird, überdacht.

Die Vorteile der Dünnschichtausführung, die unter Verwendung der Methoden der Dünnschichttechnik erreicht werden, bestehen darin, daß auf kleinstem Raum neben- oder übereinander eine beliebige Anzahl von Dünnschichtpufferbehältern 1 höchst präzise angeordnet werden kann, was für den praktischen Einsatz insofern unbedingt nötig ist, da erst dann eine exakte Zuordnung zwischen Meß- bzw. Beeinflussungsergebnis und dem Gewebeaufbau hergestellt werden kann. Durch diese Präzision ebenso wie durch die Vielzahl der Meß- und Beeinflussungsstellen wird ein neuer Effekt in der Hinsicht erzielt, daß ein Zusammenhang zwischen verschiedenen Aktivitäten und Parameteränderungen im Gewebe dargestellt und eine räumliche Auflösung oben beschriebener Vorgänge durch die Gleichzeitigkeit der Messungen bzw. Beeinflussungen aufgezeigt werden kann, wobei völlig neue Erkenntnisse gewonnen werden.

Die Auswahl der Medien 3, mit denen die Dünnschichtpufferbehälter 1 gefüllt sind, bestimmt die meß- bzw. beeinflußbaren Parameter bzw. Stoffe. Demnach kann das Medium 3 aus flüssigen und/oder festen Stoffen, etwa Elektrolyten oder Ionenaustauschern oder einer Kombination aus diesen bestehen. Ebenso bestimmend für die Art der Messung bzw. Beeinflussung ist die Auswahl der Elektroden- bzw. Geberflächenmaterialien 2, die sowohl aus Edelmetallen, vorzugsweise aus Au oder Pt, oder Metallverbindungen, vorzugsweise aus Ag/AgCl

bestehen können, sie können aber auch als Thermoelement oder Widerstandsthermometer ausgeführt sein oder derartig, daß mindestens zwei der Elektroden- bzw. Geberflächen 2 gemeinsam mit dem Medium 3 eine elektrochemische Zelle bilden. Dadurch wird unter anderem auch ein wesentlicher, neuer Effekt erreicht: die bei der polarographischen Messung fließenden Ströme fließen innerhalb des Dünnschichtpufferbehälters 1 und beeinflussen dadurch nicht in unerwünschter Weise z.B. die Gewebeaktivität.

Eine mögliche Gesamtausführung der Dünnschichtpufferbehältersonde ist in Fig. 4 im Schnitt dargestellt. Dabei wird das isolierende Substrat 7, das aus anwendungs- oder fertigungstechnischen Gründen günstigerweise zusätzlich auf einem isolierenden und/oder leitenden Zusatzsubstrat 20 aufgebracht sein kann, vorzugsweise auf einem isolierenden Trägersubstrat 13, auf dem Leiterbahnen 14 angeordnet sind, befestigt. Die Leiterbahnen 8 des isolierenden Substrates 7 werden mit den Leiterbahnen 14 des Trägersubstrates 13 elektrisch verbunden 15.

Um einfach, rasch und zuverlässig einen elektrischen Kontakt mit den Leiterbahnen 14 und 8 und damit zu den Elektroden- bzw. Geberflächen 2 in den Dünnschichtpufferbehältern 1 zu gewährleisten, werden auf dem isolierenden Trägersubstrat 13 Stecker 16 derartig befestigt, daß sie elektrisch verbunden 19 sind mit den Leiterbahnen 14.

Durch geeignetes Eingießen der beschriebenen Anordnung mit isolierendem Material 17, vorzugsweise Kunstharz od.dgl., wird durch das isolierende Material 17 und das isolierende Substrat 7 mit den darauf befindlichen Dünnschichtpufferbehältern 1 ein Behälter 18 gebildet, der nach oben offen ist und beliebig verschlossen werden kann. Diese Anordnung eignet sich besonders für Gewebekulturunterschungen, wobei die Gewebekultur in den Behälter 18 eingebracht wird.

Eine andere mögliche Gesamtausführung der Dünnschichtpufferbehältersonde zeigt Fig. 5 im Schnitt. Das isolierende Substrat 7 ist nadelförmig ausgestaltet. Für den Fall, daß das isolierende Substrat 7 auf einem isolierenden und/oder leitenden Zusatzsubstrat 20 aufgebracht ist, ist auch das Zusatzsubstrat 20 in derselben Form wie das isolierende Substrat 7 nadelförmig ausgeführt. Dadurch werden Gewebeverletzungen vermieden. Durch das isolierenede Material 17 werden die nichtisolierten Teil der Leiterbanhnen 8 und 14 sowie die elektrichen Verbindungen 15 und 19 eingegossen.

Eine weitere mögliche Gesamtausführung zeigt Fig. 6. Das isolierende Substrat 7 bzw. das isolierende und/oder leitende Zusatzsubstrat 20 sind zylinderförmig ausgeführt. Für die übrige Ausführung gilt die gleiche Beschreibung wie bei Fig. 5.

Die beiden in Fig 5 und 6 dargestellten Ausführungsformen eignen sich besonfers für Untersuchungen bzw. Beeinflussungen von tiefer gelegenen Gewebestrukturen, z. B.tiefen Gehirnregionen, subkutanen Gewebeteilen od.dgl.

Eine Variante der in Fig. 4 gezeigten Ausführung ist die in Fig. 7 dargestellte: bei geeigneter Einbettung der beschriebenen Sonde mit isolierendem Material 17 bildet das isolierende Material 17 Behälter 18, die nach oben offen sind und die Öffnung 4 der Kammer 1 frei lassen.

**Patentansprüche**

1. Medizinische Sonde in Dünnschichtausführung, insbesondere zur Registrierung bzw. Beeinflussung von Gewebeaktivitäten, mit einem isolierenden Substrat, auf welchem Leiterbahnen angeordnet sind, welche gegenüber dem Meßgut mittels einer Isolierschichtabdeckung, z.B. aus $SiO_2$, isoliert sind und zu auf dem isolierenden Substrat aufliegenden Elektroden- bzw. Geberflächen führen, dadurch gekennzeichnet, daß mindestens eine Elektroden- bzw. Geberfläche (2) von einer auf dem isolierenden Substrat (7, 11) aufgebrachten zeiten Isolierschichtabdeckung (6), die vorzugsweise eine Wandstärke von 0,5/µm bis 10 µm, insbesondere von 3 µm, aufweist, unter Bildung einer, mit einem gasförmigen und/oder flüssigen und/oder festen Medium (3) gefüllten Kammer (1) überdacht ist, die mindestens eine in Bezug auf die Größe der Kammer (1) kleine Offnung (4) aufweist, wobei als Stoffe für die Isolierschichtabdeckung (6) $SiO_2$, $SiO_x$, $Si_3N_4$, $SiO_xN_y$, $TiO_{2i}$, $Ta_2O_5$ oder mehrere dieser Stoffe eingesetzt sind.

2. Sonde nach Anspruch 1, dadurch gekennzeichnet, daß das Medium (3) einen Elektrolyten oder einen Ionenaustauscher- oder eine Kombination aus beiden darstellt.

3. Sonde nach Anspruch 1 oder 2, dadurch gekennzeichnet, daß die Kammer (1) durch die dünne Isolierschichtabdeckung (6) in der Form bestimmt, in erhabener Weise auf dem isolierenden Substrat (7) angeordnet ist oder daß die Kammer (1) durch Einätzen einer Vertiefung (10) in das isolierende Substrat (7) und Überdecken der Vertiefung (10) mit der Isolierschichtabdeckung (6) gebildet ist oder daß die Kammer (1) in Schichtbauweise ausgeführt ist, wobei in eine Isolierschicht (11), die auf dem isolierenden Substrat (7) aufgebracht ist, eine Vertiefung (12) geätzt ist, die von der zweiten Isolierschichtabdeckung (6) überdeckt Ist.

4. Sonde nach einem der Ansdrüche 1 bis 3, dadurch gekennzeichnet, daß mehrere Kammern (1) in beliebiger Anordnung auf dem flexiblen oder steifen, isolierenden Substrat (7) angeordnet sind.

5. Sonde nach einem der Ansprüche 1 bis 4, dadurch gekennzeichnet, daß die Elektroden- bzw. Geberflächen (2) in bekannter Weise aus einem Edelmetall, vorzugsweise Au oder Pt, oder aus einer Metallverbindung, vorzugsweise Ag/AgCl bestehen oder daß die Elektroden- bzw.

Geberfläche (2) aus einem Halbleiter besteht.

6. Sonde nach einem der Ansprüche 1 bis 5, dadurch gekennzeichnet, daß wenigstens zwei der Elektroden- bzw. Geberflächen (2) mit dem Medium (3) eine elektrochemische Zelle bilden oder daß die Elektroden- bzw. Geberflächen (2) ein Thermoelement bilden oder daß die Elektroden- bzw. Geberflächen (2) ein Widerstandsthermometer bilden.

7. Sonde nach einem der Ansprüche 1 bis 6, dadurch gekennzeichnet, daß das isolierende Substrat (7) auf der vom Meßgut (9) abgewandten Fläche mit einem weiteren isolierenden und/oder leitenden Zusatzsubstrat (20) verbunden ist oder direkt auf diesem aufgebracht ist.

8. Sonde nach einem der Ansprüche 1 bis 7, dadurch gekennzeichnet, daß das isolierende Substrat (7) bzw. das isolierende und/oder leitende Zusatzsubstrat (20) auf einem isolierenden Trägersubstrat (13), auf dem sich weitere Leiterbahnen (14) befinden, befestigt/und die auf dem isolierenden Substrat angeordneten Leitenbahnen (8) mit den weiteren Leiterbahnen (14) elektrisch verbunden (15) sind.

9. Sonde nach einem der Ansprüche 1 bis 6, dadurch gekennzeichnet, daß auf dem isolierenden Trägersubstrat (13) Stecker (16) befestigt sind, die mittels einer elektrischen Verbindung (19) an den weiteren Leiterbahnen (14) angeschlossen sind.

10. Sonde nach einem der Ansprüche 1 bis 9, dadurch gekennzeichnet, daß sie derart in isolierendem Material (17) eingegossen ist, daß das isolierende Material (17) gemeinsam mit dem isolierenden Substrat (7) unter Freilassung der darauf befindlichen Kammern (1) einen nach oben offenen Behälter (18) bildet oder daß sie mit isolierendem Material (17) derart überzogen ist, daß das isolierende Material (17) unter Freilassung der Öffnungen (4) der Kammern (1) einen nach oben offenen Behälter (18) bildet.

11. Sonde nach einem der Ansprüche 1 bis 10, dadurch gekennzeichnet, daß das isolierende Substrat (7), und für den Fall, daß dieses auf einem isolierenden und/oder leitenden Zusatzsubstrat (20) aufgebracht ist, auch das Zusatzsubstrat (20) in der selben Form wie das Substrat (7) nadelförmig oder zylinderförmig ausgeführt ist und durch das isolierende Matetrial (17), vorzugsweise die nichtisolierten Teile der Leiterbahnen (8,14) sowie die elektrischen Verbindungen (15, 19) eingegossen sind.

**Claims**

1. A medical probe of the thin layer type, in particular for recording and/or influencing the activity of tissue, having an insulating substrate on which strip conductors or conductor tracks which are insulated against the substance to be measured by means of an insulating layer cover, for instance of $SiO_2$, are arranged and lead to the electrode or transmitting surfaces superimposed on the insulating substrate, characterized in that at least one electrode or transmitting surface (2) is roofed over (spanned) by an insulating layer cover (6) applied to the insulating substrate (7, 11) and preferably having a wall thickness of 0,5 μm to 10 μm, in particular 3 μm, under formation of a chamber (1) filled with a gaseous and/or liquid and/or solid medium (3) having at least one opening (4) which is small in relation to the dimensions of the chamber (1), and that the substances used for the insulating layer cover (6) are $SiO_2$, $SiO_x$, $Si_3N_4$, $SiO_xN_y$, $TiO_2$, $Ta_2O_5$ or several of these substances.

2. The probe according to claim 1, wherein the medium (3) constitutes an electrolyte or an ion exchanger or a combination of the two.

3. The probe according to claim 1 or 2, wherein the chamber (1) whose form is determined by the thin insulating cover (6) is arranged in a raised manner on the insulating substrate (7) or that the chamber (1) is formed by etching a recess (10) into the insulating substrate (7) and covering the recess (10) with the insulating layer cover (6) or that the chamber (1) is constructed in stacks of layers, with a recess (12) etched into one insulating layer (11) applied to the insulating substrate (7), the recess (12) being roofed over (spanned) by the insulating layer cover (6).

4. The probe according to any one of the claims 1 to 3, wherein several chambers (1) are arranged in any given arrangement on the flexible or rigid insulating substrate (7).

5. The probe according to any one of the claims 1 to 4, wherein the electrode or transmitting surfaces (2) consist in a manner known per se of a noble metal, preferably Au or Pt, or of a metal compound, preferably Ag/AgCl, or that the electrode or transmitting surface (2) consists of a semi-conductor.

6. The probe according to any one of the claims 1 to 5, wherein at least two of the electrode or transmitting surfaces (2) form an electrochemical cell together with the medium (3) or the electrode or transmitting surfaces (2) form a resistance thermometer.

7. The probe according to any one of the claims 1 to 6, wherein the insulating substrate (7) is connected to a further insulating and/or conductive additional substrate (20) on the surface facing away from the substance (9) to be measured or directly applied to said additional substrate (20).

8. The probe according to any one of the claims 1 to 7, wherein the insulating substrate (7) or the insulating and/or conductive additional substrate (20) is fixed to an insulating carrier substrate (13) on which further strip conductors or conductor tracks (14) are provided, and the strip conductors or conductor tracks (8) provided on the insulating substrate are electrically connected (15) to the further strip conductors or conductor tracks (14).

9. The probe according to any one of the claims 1 to 6, wherein connectors (16) connected to the further strip conductors or conductor tracks (14) by means of an electrical connection (19) are fixed to the insulating carrier substrate (13).

10. The probe according to any one of the claims 1 to 9, wherein the probe is embedded in insulating material (17) in such a manner that the insulating material (17) together with the insulating substrate (7) forms a container (18) open towards the top, sparing the chambers (1) disposed thereon, or said probe is coated with insulating material (17) in such a manner that the insulating material (17) forms a container (18) open towards the top sparing the openings (4) of the chambers (1).

11. The probe according to any one of the claims 1 to 10, wherein the insulating substrate (7), and in case this is applied to an insulating and/or conductive additional substrate (20), the additional substrate (20), as well, is shaped in the same form of needles or cylinders as the substrate (7) and is embedded by means of the insulating material (17), preferably the uninsulated parts of the strip conductors or conductor tracks (8, 14) and the electrical connections (15, 19).

**Revendications >1**

1. Sonde médicale en réalisation de couche mince, en particulier pour l'enregistrement de ou l'influence sur les activités de tissus, avec un substrat isolant sur lequel sont arrangées des pistes conductives isolées par rapport à la matière à mesurer au moyen d'une couche isolante de couverture, par exemple en $SiO_2$ et mènent aux surfaces électrodes ou transmetteuses disposées sur le substrat isolant, caractérisée en ce que au moins une surface életrode ou transmetteuse (2) est coiffée par une couche isolante de couverture (6) disposée sur le substrat isolant (7, 11) et pourvue de préférence d'une épaisseur de paroi de 0,5 μm à 10 μm, en particulier de 3 μm, en formant une chambre (1) remplie d'un milieu (3) gazeux et/ou liquide et/ou solide et pourvue d'au moins un orifice (4) petit par rapport aux dimensions de la chambre (1), les matériaux de la couche isolante de couverture (6) étant le $SiO_2$, $SiO_x$, $Si_3N_4$ $SiO_xN_y$, $TiO_2$, $Ta_2O_5$ ou plusieurs desdites substances.

2. Sonde selon la revendication 1, caractérisée en ce que le milieu (3) constitue un électrolyte ou un echangeur de ions ou une combinaison des deux.

3. Sonde selon la revendication 1 ou 2, caractérisée en ce que la chambre (1) dont la forme est determinée par la couche mince isolante de couverture (6) est disposée en relief sur le substrat isolant (7) ou que la chambre (1) est formée en mordant une encoche (10) dans le substrat isolant et couvrage de l'encoche (10) au moyen de la couche isolante de couverture (6) ou que la chambre (19) est réalisée en couches, une encoche (12) coiffée par la couche isolante de couverture (6) étant prevue par morsure dans la couche isolante (11) appliquée sur le substrat isolant (7).

4. Sonde selon une des revendications 1 à 3, caractérisée en ce que plusieurs chambres (1) sont disposées de manière quelconque sur le substrat isolant (7) flexible ou rigide.

5. Sonde selon une des revedications 1 à 4, caractérisée en ce que les surfaces électrodes ou transmetteuses (2) consistent, de manière connue en soi, en un métal precieux, de préférence Au ou Pt, ou en un compose métallique, de préférence Ag/AgCl ou que la surface electrode ou transmetteuse (2) consiste en un semiconducteur.

6. Sonde selon une des revendications 1 à 6, caractérisée en ce que au moins deux des surfaces electrodes ou transmetteuses (2) forment une cellule électrochimique ensemble avec le milieu (3) ou que les surfaces électrodes ou transmetteuses (2) forment un thermocouple ou que les surfaces électrodes ou transmetteuses (2) forment un thermometre à resistance électrique.

7. Sonde selon une des revendications 1 à 6, caractérisée en ce que le substrat isolant (7) est relié sur la surface opposée à la matière à mesurer à un substrat additionnel (20) isolant et/ou conductif ou est directement disposé sur ledit substrat additionnel.

8. Sonde selon une des revendications 1 à 7, caractérisée en ce que le substrat isolant (7) ou le substrat additionnel (20 isolant et/ou conductif est fixé sur un substrat porteur isolant (13) sur lequel se trouvent des pistes conductives (14) additionnelles et que les pistes conductives (8) disposées sur le substrat isolant sont reliées électriquement aux pistes conductives (14) additionnelles.

9. Sonde selon une des revendications 1 à 6, caractérisée en ce que des fiches (16) reliées aux pistes conductives additionnelles (14) au moyen d'un raccord électrique (19) sont fixées sur le substrat isolant (13).

10. Sonde selon une des revendications 1 à 9, caractérisée en ce que elle est coulée dans du matériau isolant (17) de manière que le matériau isolant (17) forme un récipient (18) ouvert vers le haut ensemble avec le substrat isolant (7) laissant libre les chambres (19) y disposées ou qu'elle est revêtue en matériau isolant (17) de manière que le matériau isolant (17) forme un récipient (18) ouvert vers le haut en laissant libre les orifices (4) des chambres (1).

11. Sonde selon une des revendications 1 à 10, caractérisée en ce que le substrat isolant (7) et, en cas qu'il est disposé sur un substrat (20) additionnel et/ou conductif, le substrat additionnel (20) dans la meme forme comme le substrat (7), est réalisé en aiguille ou cylindrique et coulé au moyen du matériau isolant (17), de préférence les parties non-isolantes des pistes conductives (8, 14) ainsi que les raccords électriques (15, 19).

Fig.1

Fig.2

Fig.3

1

Fig. 4

Fig.5

Fig.6

0 064 970

3

Fig. 7